# EUROPÄISCHE PATENTANMELDUNG

(11) **EP 1 518 879 A2**
(43) Veröffentlichungstag der Anmeldung: **30.03.2005**
(21) Anmeldenummer: 04021318.3
(22) Anmeldetag: 08.09.2004
(51) Int. Cl.: C08G 64/00, C08G 64/02, C08G 64/20

(54) **Verfahren zur Herstellung von aliphatischen Oligocarbonatdiolen**

(30) Priorität: 19.09.2003 DE 10343471
(71) Anmelder: Bayer MaterialScience AG, 51368 Leverkusen (DE)
(72) Erfinder: Hofacker, Steffen, Dr., 51519 Odenthal (DE); Bachmann, Rolf, Dr., 51469 Bergisch Gladbach (DE); Bäcker, Lothar, 06773 Gossa (DE); Witossek, Herbert, Dr., 04157 Leipzig (DE)

(57) **Zusammenfassung**

Die vorliegende Erfindung betrifft ein verbessertes Verfahren zur Herstellung von aliphatischen Oligocarbonatdiolen durch Umesterung von Dimethylcarbonat (DMC) mit aliphatischen Diolen.

## Beschreibung

Die vorliegende Erfindung betrifft ein verbessertes Verfahren zur Herstellung von aliphatischen Oligocarbonatdiolen durch Umesterung von Dimethylcarbonat (DMC) mit aliphatischen Diolen.

Oligocarbonatdiole können prinzipiell aus aliphatischen Polyolen durch Umsetzung mit Phosgen, bis-Chlorkohlensäureestern, Diarylcarbonaten, cyclischen Carbonaten oder Dialkylcarbonaten hergestellt werden. Sie sind wichtige Vorprodukte zur Herstellung von Kunststoffen, Lacken und Klebstoffen. Sie werden z.B. mit Isocyanaten, Epoxiden, (cyclischen) Estern, Säuren oder Säureanhydriden umgesetzt.

DE-A 101 30 882 beschreibt einen zweistufigen Prozess zur Herstellung von Oligocarbonatdiolen, bei dem zunächst bei einem Druck von 1,5 bis 100 bar und einer Temperatur von 100°C bis 300°C Dimethylcarbonat (DMC) mit einem oder mehreren aliphatischen Diolen umgesetzt wird, wobei das bei der Reaktion gebildete Methanol zusammen mit DMC als Gemisch aus der Reaktion entfernt wird. Im zweiten Schritt erfolgt die Entkappung der endständigen Hydroxylgruppen durch Anlegen von Drücken von 1 bis 1000 mbar und Temperaturen von 160°C bis 250°C für mehrere Stunden. Die bevorzugte Reaktionstemperatur für den Entkappungsschritt liegt bei 200°C und der Druck bei 100 bis 200 mbar. Je nach Variante beträgt die Verweilzeit der Reaktionsmischung bei 200°C zwischen 9 und 50 Stunden. Die so hergestellten Oligocarbonatdiole weisen bei einem zahlenmittleren Molekulargewicht Mₙ von 2000 g/mol eine OH-Zahl (OHZ) von rund 56 mg KOH/g auf. Die tatsächliche OH-Funktionalität der so erhaltenen Produkte weicht jedoch vom theoretischen Wert von 2,00 ab. Grund hierfür ist die Bildung von Nebenprodukten mit unerwünschten Endgruppen, welche die Funktionalität erniedrigen, z.B. Methylester-, Methylether-, Vinyl-Gruppen und anderen.

Bei vielen Folgeanwendungen, in denen Oligocarbonatdiole eingesetzt werden ist neben der OHZ die tatsächliche OH-Funktionalität (f_{OH}) und vor allem deren Konstanz von besonderer Bedeutung. Weicht die Funktionalität um mehr als 0,10 Punkte vom theoretischen Wert 2,00 ab, so führt dies aufgrund der Anteile an monofunktionellen Oligocarbonaten, die in Polymerisationsreaktionen als Kettenabbrecher fungieren, zu Werkstoffen mit deutlichen verschlechterten mechanischen Eigenschaften. Daher ist es notwendig, die tatsächliche OH-Funktionalität konstant und nahe dem theoretischen Wert von 2,00 für bifunktionelle Oligocarbonatpolyole zu halten.

Aufgabe der Erfindung war es daher, ein verbessertes Verfahren zur Herstellung von Oligocarbonatdiolen auf Basis von Dimethylcarbonat zur Verfügung zu stellen, welches zu Oligocarbonatdiolen mit einer OH-Funktionalität ≥1,90 führt.

Es wurde gefunden, dass dies durch kontinuierliches Zudosieren von DMC bei gleichzeitiger schneller Entfernung von Methanol aus dem Reaktionsgemisch und Kontrolle der Umsetzungstemperatur während des Entkappungsschrittes erreicht werden kann.

Gegenstand der Erfindung ist ein Verfahren zur Herstellung von Oligocarbonatdiolen, bei dem in einem ersten Schritt Dimethylcarbonat mit einer Diolkomponente, die ein aliphatisches Diol oder ein Gemisch von aliphatischen Diolen oder ein Gemisch von aliphatischen Diolen mit Lactonen sein kann, unter Zusatz eines Umesterungskatalysators bei einem Druck von 3 bis 6 bar und bei Temperaturen von 100 bis 200°C, bevorzugt 130 bis 200°C, besonders bevorzugt 140 bis 200°C umgesetzt werden, wobei das DMC kontinuierlich in die flüssige Phase des Reaktionsgemisches eingeleitet und das entstehende Methanol in Form eines gasförmigen Methanol/Dimethylcarbonat-Gemisches durch Destillation kontinuierlich aus dem Reaktionsgemisch entfernt wird, und in einem zweiten Schritt nach Beendigung der kontinuierlichen Zudosierung von DMC zunächst die Temperatur des Reaktionsgemisches auf ≤190°C, bevorzugt ≤180°C abgesenkt wird und danach der Druck langsam auf Normaldruck erniedrigt wird, wobei parallel hierzu weiteres Methanol/Dimethylcarbonat-Gemisch abdestilliert wird, nach Erreichen des Normaldrucks der Druck schrittweise weiter verringert und nach Erreichen eines Drucks ≤100 mbar in das Reaktionsgemisch ein Inertgasstrom eingeleitet wird.

Der zusätzliche Bedarf an DMC aufgrund des destillativen Verlustes während der Umesterungsphase beträgt 5 bis 25%, bevorzugt 5 bis 20%, bezogen auf die stöchiometrisch notwendige Menge. Durch die kontinuierliche Zugabe von DMC und die unmittelbare Entfernung des Methanols aus dem Reaktionsgemisch bei einer Umesterungstemperatur von 100 bis 200°C und einem Druck zwischen 3 bis 6 bar wird gewährleistet, dass die Nebenproduktbildung von Methyletherendgruppen stark vermindert bzw. vermieden werden kann, was zu einer Erhöhung der OH-Funktionalität beiträgt.

Dabei ist es erfindungswesentlich, dass die DMC-Konzentration im Reaktionsgemisch so eingestellt wird, dass einerseits die Nebenproduktbildung und der erforderliche DMC-Überschuss minimiert werden und andererseits die Raum-Zeit-Ausbeute nicht beeinträchtigt wird. Dies geschieht bevorzugt durch Konstanthalten des Drucks und der Temperatur, wodurch (temperaturabhängig) ein bestimmter Partialdruck des Dimethylcarbonates eingestellt wird. Der Gesamtdruck wird bevorzugt durch Einleiten von Inertgas in den Reaktor aufgebaut. Wichtig ist, dass die Zugabe des DMC derart erfolgt, dass der sich einstellende Eigendruck des DMC nicht den vorgegebenen Gesamtdruck übersteigt. Somit wird gewährleistet, dass sowohl die Nebenproduktbildung stark vermindert als auch der erforderliche stöchiometrische Überschuss an DMC minimiert werden kann. Darüber hinaus wird es aufgrund kontinuierlicher Druckregelung bei konstanter Umesterungstemperatur und kontrollierter Zudosierung von DMC möglich, ohne nachträgliche Korrekturen der Stöchiometrie (z.B. durch Zugabe von weiterem DMC oder Diol/Diolgemisch) ein Oligocarbonatdiol, mit der gewünschten Molmasse bzw. Molmassenverteilung herzustellen.

Im zweiten erfindungsgemäßen Verfahrensschritt (Entkappung) wird nach Beendigung der kontinuierlichen Zudosierung von DMC zunächst die Temperatur des Reaktionsgemisches auf ≤190°C, bevorzugt ≤180°C abgesenkt. Danach wird der Druck langsam auf Normaldruck zurückgenommen, wobei parallel hierzu weiteres Methanol/DMC-Gemisch abdestilliert wird. Nach Erreichen des Normaldrucks wird der Druck schrittweise weiter verringert. Bei Erreichen eines Drucks ≤100 mbar wird in das Reaktionsgemisch ein Inertgasstrom eingeleitet. Dieser bewirkt neben den Vakuumbedingungen ein zusätzliches Herausschleppen von sich bildendem Methanol bzw. noch vorhandenem DMC. Durch dieses Vorgehen wird die Entstehung von zusätzlichen endständigen Hydroxylgruppen begünstigt, was zu einer Erhöhung der OH-Funktionalität und damit zu einer verbesserten Produktqualität beiträgt.

Als Inertgase kommen solche zum Einsatz, die in den chemischen Prozess nicht eingreifen. Beispiele sind Stickstoff, Argon, Methan und Erdgas. Bevorzugt wird Stickstoff eingesetzt.

Ein wesentliches Merkmal des Entkappungsschrittes liegt in der Temperaturkontrolle dieser Phase. Wird die Reaktionstemperatur auf mehr als 190°C gesteigert, so kommt es vermehrt zu Nebenproduktbildung. Im wesentlichen entstehen hierbei endständige Vinylgruppen, die zu einer drastischen Erniedrigung der OH-Funktionalität führen. Daher ist es für das erfindungsgemäße Verfahren wesentlich, die Temperatur während der Entkappungsphase auf Werte ≤190°C, bevorzugt ≤180°C einzustellen. So lässt sich die Bildung von endständigen Vinylgruppen vermeiden und damit die OH-Funktionalität erhöhen.

Nach einer Haltezeit zwischen 10 und 50 Stunden bei einem Druck ≤100 mbar und einer Temperatur ≤190°C, bevorzugt ≤180°C, wird der Druck durch Belüftung mit Inertgas, bevorzugt Stickstoff, auf Normaldruck erhöht. Anschließend können Produkteigenschaften wie OH-Zahl, Viskosität, zahlenmittleres Molekulargewicht, OH-Funktionalität usw. bestimmt werden.

Ist das zahlenmittlere Molekulargewicht des Produktes nach der Entkappungsphase zu hoch, so wird es durch Zugabe entsprechender Mengen Diol und/oder Diolmischung und erneutes Aufheizen des Reaktionsgemischs auf eine Temperatur von 100 bis 200°C (analog dem ersten Verfahrensschritt) für ein bis fünf Stunden korrigiert. Ein erneuter Entkappungsschritt ist nach dieser Korrektur nicht zwingend notwendig, sofern vor der Korrektur bereits alle endständigen OH-Gruppen frei vorlagen und durch Zugabe der Diol-Komponente keine erneute Verkappung der endständigen Hydroxylgruppen aufgebaut wird.

Ist das zahlenmittlere Molekulargewicht des Produktes nach der Entkappungsphase zu niedrig, so wird es durch Zugabe entsprechender Mengen von DMC und erneutes Aufheizen des Reaktionsgemisches auf eine Temperatur von 100 bis 200°C (analog dem ersten Verfahrensschritt) für ein bis fünf Stunden korrigiert. Da dieses Vorgehen zu einem erneuten Aufbau der Verkappung führt, schließt sich ein erneuter Entkappungsschritt (analog dem zweiten Verfahrensschritt) an. Die Laufzeiten der erneuten Entkappung können jedoch aufgrund der verhältnismäßig kleinen zugesetzten DMC-Korrekturmenge stark reduziert werden. Wesentlich ist jedoch auch hier die Kontrolle der Temperatur analog dem oben beschrieben zweiten Verfahrensschritt.

Als Umesterungskatalysatoren des erfindungsgemäßen Verfahrens kommen Titan-Verbindungen (z.B. Titantetraisopropylat, Titantetrabutylat etc.) oder Ytterbium-Verbindungen (z.B. Ytterbium(III)heptadionat, Ytterbium(III)acetylacetonat, etc.) oder Mischungen daraus zum Einsatz. Bevorzugt werden Titantetraisopropylat oder/und Ytterbium(III)acetylacetonat eingesetzt. Besonders bevorzugt wird Titantetraisopropylat eingesetzt.

Die eingesetzten Katalysatorgehalte betragen 0,01 bis 1000 ppm, bevorzugt 0,1 bis 500 ppm, besonders bevorzugt 1 bis 200 ppm, bezogen auf die Gesamtmenge des hergestellten Oligocarbonatdiols. Bei Erreichen der vorgegebenen Kennzahl werden, wenn Titan-Verbindungen als Katalysator eingesetzt werden, diese durch Zusatz der 1- bis 2-fachen Gewichtsmenge Dibutylphosphat, bezogen auf die Menge des eingesetzten Katalysators deaktiviert. Bei Einsatz von Ytterbium-Verbindungen als Katalysator kann auf eine Deaktivierung verzichtet werden. Eine anschließende Maskierung, Fällung oder sonstige Entfernung oder Deaktivierung ist nicht notwendig.

Im erfindungsgemäßen Verfahren wird eine Diolkomponente eingesetzt, die ein aliphatisches Diol oder ein Gemisch von aliphatischen Diolen oder ein Gemisch von aliphatischen Diolen mit Lactonen ist.

Es können aliphatische Diole mit 4 bis 50 C-Atomen in der Kette (verzweigt oder unverzweigt), die auch durch zusätzliche Heteroatome wie Sauerstoff (O), Schwefel (S) oder Stickstoff (N) unterbrochen sein können, eingesetzt werden. Beispiele geeigneter Diole sind 1,4-Butandiol, 1,5-Pentandiol, 1,6-Hexandiol, 1,7-Heptandiol, 1,12-Dodecandiol, Cyclohexandimethanol, 3-Methyl-1,5-Pentandiol, 2,4-Diethyl-1,5-Pentandiol, Bis-(2-hydroxyethyl)-ether, Bis-(6-hydroxyhexyl)-ether, durch Reduktion von dimerisierten ungesättigten Fettsäuren hergestellte Diole ("Dimerdiole") oder kurzkettige C₂-, C₃-, oder C₄-Polyetherdiole mit einem zahlenmittleren Molekulargewicht < 700 g/mol sowie Mischungen daraus.

Weiterhin können die Additionsprodukte der Diole mit Lactonen (Esterdiole), z.B. ε-Caprolacton, Valerolacton etc., sowie Mischungen der Diole mit Lactonen eingesetzt werden wobei eine einleitende Umesterung von Lacton und Diol nicht nötig ist.

Bevorzugt werden im erfindungsgemäßen Verfahren Mischungen aus 1,6-Hexandiol und/oder 1,5-Pentandiol und/oder 1,4-Butandiol und ε-Caprolacton und/oder Valerolacton eingesetzt, ganz besonders bevorzugt Mischungen aus 1,6-Hexandiol und ε-Caprolacton.

Das erfindungsgemäße Verfahren ermöglicht die Herstellung von Oligocarbonatdiolen, Oligocarbonatesterdiolen, Oligocarbonatetherdiolen oder Mischungen daraus mit zahlenmittleren Molekulargewichten von 500 bis 5000 g/mol, bevorzugt 500 bis 2000 g/mol, einer OH-Funktionalität von 1,85 bis 2,00, bevorzugt 1,90 bis 2,00 und einem Restgehalt an Methanol und DMC von jeweils < 0,10 mol-%, und Gehalten an endständigen Vinylgruppen < 0,10 mol-% und endständigen Methylethergruppen < 5,0 mol-%. Die angegebenen mol-%-Gehalte können als Anteile der aufgeführten Verbindung bezogen auf 1 mol der theoretischen Zielverbindung mit zwei endständigen Hydroxylgruppen angesehen werden.

Die nach dem erfindungsgemäßen Verfahren hergestellten Oligocarbonatdiole können mit Isocyanaten, Epoxiden, (cyclischen) Estern, Säuren oder Säurenhydriden umgesetzt und damit beispielsweise zur Herstellung von Polymeren, Kunststoffen, Fasern, Beschichtungen, Lacken und Klebstoffen eingesetzt werden. Ebenso können die erfindungsgemäß hergestellten Oligocarbonatdiole als Bestandteile in polyurethanbasierten Gießelastomeranwendungen verwendet werden. Darüber hinaus eignen sie sich als Bausteine für feuchtigkeitshärtende Beschichtungen, als Bindemittel oder Bindemittelbestandteile und/oder Reaktivverdünner in lösemittelhaltigen oder wässrigen Polyurethanbeschichtungen. Sie können weiterhin als Bausteine für freie NCO-Gruppen enthaltende Polyurethanprepolymere oder in Polyurethandispersionen oder Polyurethanlösungen eingesetzt werden. Die nach dem erfindungsgemäßen Verfahren hergestellten Oligocarbonatdiole können auch zur Herstellung thermoplastischer Kunststoffe wie aliphatischer und/oder aromatischer Polycarbonate, thermoplastischer Polyurethane etc. eingesetzt werden.

### Beispiele

Die in den Beispielen aufgeführten Gehalte an Verbindungen, die abweichend von der theoretischen Zielverbindung nur eine oder keine endständigen Hydroxylgruppen tragen, wurden ebenso wie das zahlenmittlere Molekulargewicht über ¹H-NMR-Analytik und die integrale Auswertung der entsprechenden Signale ermittelt. Die in mol-% angegebenen Gehalte können als Anteile der aufgeführten Verbindung bezogen auf 1 mol der theoretischen Zielverbindung mit zwei endständigen Hydroxylgruppen angesehen werden. Bei der Berechnung der OH-Funktionalität werden die so ermittelten Werte als Anteile von Kettenabbrechermolekülen aufgefasst und entsprechend berücksichtigt.

### Beispiel 1

9270 kg 1,6-Hexandiol und 8950 kg ε-Caprolacton wurden bei 70°C im gerührten Reaktor mit Kolonne und Totalkondensator vorgelegt. 1,5 kg Titantetraisopropylat wurden zugegeben. Anschließend wurde der Kesseldruck durch Einleiten von Stickstoff auf 5,2 bar absolut erhöht und konstant geregelt und der Kesselinhalt wurde auf 200°C aufgeheizt. Innerhalb von 15 h wurden 7300 kg Dimethylcarbonat zudosiert. Gleichzeitig wurde entstehendes Methanol mit Anteilen an Dimethylcarbonat abdestilliert. Nach Ende der Zugabe wurde 0,5 h nachgerührt. Anschließend wurde die Temperatur auf 180°C erniedrigt und der Reaktordruck innerhalb von 3 h auf Normaldruck abgesenkt.

Der Reaktordruck wurde dann innerhalb von 12 h auf 90 mbar absolut abgesenkt. Nach Erreichen von 90 mbar wurden 2 m³/h Stickstoff über ein Durchleitrohr in die Reaktionsmischung eingeleitet, um restliches Methanol zu entfernen; weiterhin wurde das Vakuum bis ca. 30 mbar abgesenkt. Der Ansatz wurde insgesamt 26 h bei einer Temperatur von 180°C und einem Druck von < 60 mbar gerührt. OH-Zahl und Viskosität wurden alle 4 h ermittelt. Die OH-Zahl des Produkts wurde durch Zugabe von insgesamt ca. 250 kg 1,6-Hexandiol auf den Zielwert korrigiert. Nach Ablauf der Gesamt-Entkappungszeit wurden 2,0 kg Dibutylphosphat in den Reaktionsansatz eingerührt. Anschließend wurde der Reaktor belüftet und die Reaktionsmischung auf 100°C abgekühlt. Man erhielt 20000 kg eines klaren, farblosen, bei Raumtemperatur nicht kristallisierenden Harzes mit folgenden Kennzahlen:

| | |
|---|---|
| zahlenmittleres Molgewicht | 2000 g/mol |
| OH-Zahl | 57,8 mg KOH/g |
| Viskosität bei 23°C | 15800 mPas |
| Farbzahl (APHA) | 60 Hazen |
| OH-Funktionalität | 1,94 |
| Gehalt an endständigen Vinylgruppen | 0,0 mol-% |
| Gehalt an endständigen Methylethergruppen | 1,7 mol-% |

### Vergleichsbeispieil 1

In einem Rührkessel wurden 67,1 kg ε-Caprolacton, 69,5 kg 1,6-Hexandiol, 30,2 kg Dimethylcarbonat sowie 11 g Titantetraisopropylat vorgelegt. Nach zweimaligem Inertisieren durch Anlegen von Vakuum und anschließendem Belüften mit Stickstoff wurde der Ansatz auf 140°C aufgeheizt. Der Druck wurde mit Stickstoff auf 5,2 bar absolut erhöht und mit Hilfe einer Druckregelung konstant geregelt. Unter Rückflussbedingungen wurde die Temperatur innerhalb von 2 h auf 194°C erhöht und 1 h unter diesen Bedingungen gehalten. Danach wurde der Übergang zur Vorlage geöffnet und ein aus Methanol und DMC bestehendes Destillat abdestilliert. Nach zweistündiger Destillation wurden über ein Tauchrohr weitere 30,2 kg DMC innerhalb von 7 h bei einer Temperatur zwischen 186 und 192°C zudosiert. Gleichzeitig wurde ein aus Methanol und DMC bestehendes Destillat über Kopf in eine Vorlage abdestilliert. Nach Ende der DMC-Zugabe wurde innerhalb von 1 h die Temperatur zunächst auf 196°C erhöht und es wurde bei diesen Bedingungen 4 h nachgerührt. Anschließend wurde die Temperatur innerhalb von 1 h auf 200°C erhöht und es wurde 2 h nachgerührt.

Innerhalb von 1 h wurde der Druck auf Normaldruck (1040 mbar), anschließend innerhalb von 4 h auf 100 mbar abgesenkt. Die Temperatur wurde weiterhin konstant bei 200°C belassen. Nach 17 h erhielt man ein Reaktionsprodukt mit einer OH-Zahl von 35,1 mg KOH/g und einer Viskosität von 50000 mPas. Nach Zugabe von insgesamt 3 kg 1,6-Hexandiol bei 140°C und Normaldruck wurde die Temperatur auf 200°C erhöht, der Druck auf 100 mbar erniedrigt und so das Reaktionsgemisch weitere 10 h entkappt. Nach erneuter Zugabe von 2 kg 1,6-Hexandiol unter o.a. Zugabebedingungen wurde bei 200°C und 100 mbar weitere 30 h entkappt. Nach Abkühlen auf 80°C und Belüften mit Stickstoff wurden 14 g Dibutylphosphat in die Reaktionsmischung eingerührt. Man erhielt ein Reaktionsprodukt mit folgenden Kennzahlen:

| | |
|---|---|
| zahlenmittleres Molgewicht | 1880 g/mol |
| OH-Zahl | 60,3 mg KOH/g |
| Viskosität bei 23°C | 13000 mPas |
| Farbzahl (APHA) | 8 Hazen |
| OH-Funktionalität | 1,88 |
| Gehalt an endständigen Vinylgruppen | 4,0 mol-% |
| Gehalt an endständigen Methylethergruppen | 1,9 mol-% |

### Vergleichsbeispiel 2

In einem Rührkessel wurden 192,4 kg ε-Caprolacton, 199,2 kg 1,6-Hexandiol, 86,7 kg Dimethylcarbonat sowie 32 g Titantetraisopropylat vorgelegt. Nach zweimaligem Inertisieren durch Anlegen von Vakuum und anschließendem Belüften mit Stickstoff wurde der Ansatz auf 140°C aufgeheizt. Anschließend wurde der Druck mit Stickstoff auf 5,2 bar absolut erhöht und mit Hilfe einer Druckregelung konstant geregelt. Unter Rückflussbedingungen wurde die Temperatur innerhalb von 2 h auf 194°C erhöht und 1 h gehalten. Danach wurde der Übergang zur Vorlage geöffnet und ein aus Methanol und DMC bestehendes Destillat abdestilliert. Nach 2 h Destillation wurden über ein Tauchrohr innerhalb von 3 h weitere 86,7 kg DMC bei einer Temperatur von 194°C zudosiert. Gleichzeitig wurde ein aus Methanol und DMC bestehendes Destillat über Kopf in eine Vorlage abdestilliert. Nach Ende der Zugabe wurde die Temperatur zunächst auf 196°C erhöht und es wurde bei diesen Bedingungen 3 h nachgerührt. Anschließend wurde die Temperatur innerhalb von 0,5 h auf 200°C erhöht und es wurde 2 h nachgerührt.

Innerhalb von 1,5 h wurde der Druck auf Normaldruck (1040 mbar) und anschließend in 4 h auf 135 mbar, innerhalb weiterer 8 h auf 100 mbar abgesenkt. Die Temperatur wurde weiterhin konstant bei 200°C belassen. Nach weiteren 40 h Entkappungszeit bei 100 mbar und 200°C wurde der Ansatz auf 80°C abgekühlt und mit Stickstoff belüftet. Schließlich wurden 42 g Dibutylphosphat in die Reaktionsmischung eingerührt. Man erhielt ein Reaktionsprodukt mit folgenden Kennzahlen:

| | |
|---|---|
| zahlenmittleres Molgewicht | 1920 g/mol |
| OH-Zahl | 53,3 mg KOH/g |
| Viskosität bei 23°C | 15000 mPas |
| Farbzahl (Apha) | 477 Hazen |
| OH-Funktionalität | 1,79 |
| Gehalt an endständigen Vinylgruppen | 2,5 mol-% |
| Gehalt an endständigen Methylethergruppen | 3,4 mol-% |

## Patentansprüche

1. Verfahren zur Herstellung von Oligocarbonatdiolen, bei dem in einem ersten Schritt Dimethylcarbonat mit einer Diolkomponente unter Zusatz eines Umesterungskatalysators bei einem Druck von 3 bis 6 bar und bei Temperaturen von 100 bis 200°C umgesetzt werden, wobei das DMC kontinuierlich in die flüssige Phase des Reaktionsgemisches eingeleitet und das entstehende Methanol in Form eines gasförmigen Methanol/Dimethylcarbonat-Gemisches durch Destillation kontinuierlich aus dem Reaktionsgemisch entfernt wird, und in einem zweiten Schritt nach Beendigung der kontinuierlichen Zudosierung von Dimethylcarbonat zunächst die Temperatur des Reaktionsgemisches auf ≤190°C abgesenkt wird und danach der Druck auf Normaldruck erniedrigt wird, wobei parallel hierzu weiteres Methanol/Dimethylcarbonat-Gemisch abdestilliert wird, nach Erreichen des Normaldrucks der Druck weiter verringert und nach Erreichen eines Drucks ≤100 mbar in das Reaktionsgemisch ein Inertgasstrom eingeleitet wird.

2. Verfahren gemäß Anspruch 1, bei dem als Umesterungskatalysator Titantetraisopropylat eingesetzt wird.

3. Verfahren gemäß Anspruch 1, bei dem als Umesterungskatalysator Ytterbium(III)acetylacetonat eingesetzt wird.

4. Verfahren gemäß einem der Ansprüche 1 bis 3, bei dem die Diolkomponente ein Gemisch aus 1,6-Hexandiol und ε-Caprolacton ist.
